Europäisches Patentamt

European Patent Office

Office européen des brevets

(19)

(11) Numéro de publication : **0 086 704**
**B1**

(12) **FASCICULE DE BREVET EUROPÉEN**

(45) Date de publication du fascicule du brevet :
07.05.86

(21) Numéro de dépôt : 83400263.6

(22) Date de dépôt : 08.02.83

(51) Int. Cl.⁴ : **B 01 J   2/26**, B 01 J   2/02,
A 61 K   9/16, A 61 J   3/06

(54) Procédé et appareil pour la production de granules parfaitement sphériques et poreux, et granules ainsi obtenus.

(30) Priorité : 15.02.82 CH 916/82

(43) Date de publication de la demande :
24.08.83 Bulletin 83/34

(45) Mention de la délivrance du brevet :
07.05.86 Bulletin 86/19

(84) Etats contractants désignés :
AT BE CH DE GB IT LI LU NL SE

(56) Documents cités :
DE-C-   475 555
FR-A- 2 043 230
GB-A- 1 182 124
US-A- 3 598 373
US-A- 3 933 679

(73) Titulaire : **MICROENCAPSULATION S.A. Société dite:**
**35 Corso San Gottardo**
**Chiasso Tessin (CH)**

(72) Inventeur : **Scapinelli, Bruno**
**5 Allée Guynemer Domaine de la Maisonnière**
**F-60260 Lamorlaye (FR)**

(74) Mandataire : **Ores, Irène et al**
**CABINET ORES 6, Avenue de Messine**
**F-75008 Paris (FR)**

Jouve, 18, rue St-Denis, 75001 Paris, France

EP 0 086 704 B1

## Description

La présente invention est relative à un procédé et à un appareil pour la production de granules parfaitement sphériques et à porosité élevée.

Des processus d'encapsulation ou de formation de granule par projection de gouttes d'un liquide sur un lit de matière pulvérulente, sont déjà connus. Ces procédés, employés surtout dans l'industrie pharmaceutique, ne permettent pas d'obtenir des granules parfaitement sphériques ou poreux tels qu'ils sont nécessaires pour de nombreuses applications, par exemple pour les médicaments à libération contrôlée et/ou à action prolongée, pour les produits homéopathiques et de nombreuses autres utilisations de tels granules.

En effet, dans ces procédés, connus dans l'Art antérieur, on n'avait pas résolu le problème du contrôle étroit des dimensions des caractéristiques physiques et de la forme géométrique des granules, qui est, en fait, fondamental quand on veut obtenir des granules aptes aux applications susmentionnées.

C'est en effet le cas du brevet allemand 475 555, qui vise à transformer des matières pulvérulentes, en petits éléments uniformes qui peuvent réagir avec des gaz : selon ce brevet, on laisse tomber une à une sur la surface d'une couche de poudre, des gouttes de liquide, tel que de l'eau ou une autre solution, puis l'on sépare par tamisage, les éléments sphériques qui se forment et on les sèche. Un tel procédé ne permet pas un contrôle strict des dimensions et de la forme des granules obtenus, non plus d'ailleurs, que le procédé proposé par le brevet américain 3 933 679, qui vise à l'obtention de micro-particules sphériques d'oxydes, de carbures ou de nitrures minéraux, par dispersion d'un sol liquide de la matière minérale, sous forme de gouttelettes qui se solidifient par contact avec un fluide gélifiant, les gouttelettes gélifiées, solidifiées, étant ensuite frittées pour fournir les particules recherchées.

La présente invention a précisément pour but de résoudre ce problème essentiel, qui est résolu selon le procédé de l'invention, en contrôlant la forme et la dimension de la goutte émise individuellement par des moyens de distribution, au moyen d'un flux d'air laminaire et concentrique à la goutte et qui effleure la surface de la goutte à sa sortie desdits moyens.

Sur la base de ce principe général prévu par le procédé de la présente invention, les moyens pour la mise en œuvre de ce procédé pourront être constitués par une grande variété d'appareillages et un appareil donné à titre d'exemple non limitatif sera décrit dans ce qui va suivre comme représentant actuellement le mode de réalisation préféré, qui sera décrit de façon détaillée dans ce qui va suivre après avoir exposé les principes physiques qui sont à la base de la solution représentée par le procédé conforme à l'invention. Il est, en effet essentiel de bien clarifier les postulats théoriques du procédé, pour pouvoir comprendre mieux dans toute leur portée les progrès que l'on peut obtenir par son application.

Soit une matière première micronisée quelconque et un solvant volatil. Si on dépose à la surface de la matière première une goutte dudit solvant on peut considérer que les paramètres physiques suivants entrent en jeu :

a) la tension superficielle pour laquelle le liquide tend à maintenir la forme sphérique d'origine.

b) la capillarité de la matière première micronisée. Le liquide tend à migrer à travers les espaces interstitiels en direction normale à la surface de contact.

c) la viscosité du liquide qui s'oppose à la déformation de la goutte.

d) la formation d'un front interfacial entre la matière première et la surface de la goutte constituée par la solution saturée. La vitesse de ce front qui a pour origine le centre de la goutte comme sens, s'additionne à la vitesse de migration capillaire.

Le phénomène est évidemment très complexe, car la dynamique d'un tel système est extrêmement variable et dépend des paramètres du liquide de la matière première et des rapports entre eux. A la fin du procédé, on obtiendra un granule de forme concave/convexe variable.

Le procédé selon la présente invention est caractérisé par le fait que la forme et la dimension des gouttes émises individuellement sont contrôlées par un flux d'air laminaire et concentrique à la goutte, qui effleure la surface de la goutte pendant qu'elle est émise.

L'observation sur laquelle est basé le procédé conforme à la présente invention est que le système se simplifie énormément à condition que la goutte du solvant soit complètement entourée par la matière première micronisée.

En effet, chaque phénomène de migration capillaire centrifuge s'annule car toutes les forces responsables d'un tel phénomène sont radiales et contraires (car dans le cas contraire, il se formerait un vide au centre de la goutte).

Il reste donc seulement le phénomène centripète de pénétration du front saturé. Au fur et à mesure que ce front avance vers le centre, la viscosité du liquide augmente et ceci provoque la consolidation de la goutte. La conjonction de ces deux phénomènes permet l'obtention, après tamisage et évaporation du solvant, d'un granule parfaitement sphérique. Si la goutte d'origine était composée uniquement de solvant, on aurait un granule à 100° de matière première ; si la goutte était composée d'une solution d'une résine, on aurait un granule à matrice (comme le granule à diffusion usuel). Si à la place de la matière première pure il y a un mélange de la matière première avec un excipient compatible avec les exigences de solubilité (en utilisant également, le cas échéant, des mélanges de solvants pour les gouttes), on pourra obtenir des granules à composition complexe. De la même façon on pourra obtenir des associations de matière première. Les possibilités sont théoriquement très variées ; par exemple : en modifiant la méthode et en produisant des gouttes composées de solution de matière première/solvant/résine et en laissant tomber ces gouttes sur une résine micronisée d'enrobage, on provoquera la formation d'une membrane et donc on aura une microencapsulation. Il est donc possible en principe d'obtenir en une seule opération, un granule sphérique à matrice complexe avec une membrane protectrice et/ou retardatrice.

En appliquant cette méthode on pourra observer les deux phénomènes suivants :

Le premier est qu'on obtiendra un granule d'autant plus gros à partir de la goutte d'origine que la matière première sera plus soluble (les raisons sont évidentes) et il sera d'autant plus poreux que la solubilité sera plus faible. Cette dernière particularité de la technique sera précisée plus loin. Le problème général que l'on a résolu est celui du contrôle individuel des gouttes, aussi bien de leur forme que de leur dimension, au moyen d'un flux d'air laminaire qui enveloppe la goutte et effleure sa surface à sa sortie des moyens distributeurs de gouttes. On a dû, pour ce faire, résoudre en premier lieu trois problèmes : l'obtention de gouttes présentant le diamètre désiré et des dimensions égales, la possibilité d'effectuer leur contrôle visuel et la possibilité d'éviter la superposition des gouttes en train de tomber, sur des granules déjà formés. Pour le premier problème il n'est pas suffisant de réduire ou d'augmenter le diamètre de l'aiguille de sortie. En faisant ainsi, on peut obtenir des gouttes de petite dimension, mais pour certains liquides la vitesse de sortie est trop faible. La solution proposée, conformément à l'invention, consiste à utiliser un flux d'air le plus laminaire possible qui effleure la surface de la goutte à sa sortie. Ce flux, s'il est bien centré, provoque une pression sur la goutte qui s'ajoute à la force de gravitation. Ainsi, si l'on fait varier la pression de l'air (et donc la vitesse de la sortie) on peut obtenir des gouttes du diamètre voulu.

Le deuxième problème est résolu par l'emploi d'une lampe stroboscopique. On peut visualiser les gouttes à l'aide de cette dernière, si bien qu'à l'œil nu on a l'impression d'avoir un flux continu de liquide.

Le troisième problème a été résolu en faisant écouler la matière première sur une bande transporteuse appropriée, à une vitesse telle que chaque goutte tombe sur de la matière première nouvelle, cette bande transporteuse servant aussi à alimenter la matière première et à envoyer les granules qui viennent d'être formés vers la sélection et la décharge.

L'appareil préféré pour la mise en œuvre du procédé selon la présente invention, comprend en substance des moyens pour l'alimentation en matière première, des moyens pour créer et déterminer l'écoulement d'un lit ou tapis de matière première au-dessous des moyens distributeurs de gouttes d'un liquide producteur de gouttes, des moyens d'alimentation en liquide producteur de gouttes, des moyens d'alimentation et d'émission d'air sous pression autour des moyens distributeurs de gouttes, des moyens pour sélectionner et transporter les granules produits et des moyens pour recycler la matière première et les granules refusés par les moyens de sélection.

Les granules obtenus en mettant en œuvre le procédé de la présente invention sont caractérisés par leur sphéricité parfaite et leur porosité élevée qui permet une dissolution rapide du granule quand il est ingéré.

Un des meilleurs emplois du procédé de la présente invention est pour la production des neutres et en particulier de neutres homéopathiques à base de saccharose et lactose.

Les fabricants de produits homéopathiques sont en effet depuis longtemps à la recherche de granules neutres présentant des caractéristiques spécifiques que l'on ne peut pas obtenir avec les méthodes actuellement connues.

En effet, les neutres actuellement disponibles présentent une porosité très faible et un temps de dissolution suslinguale assez long (environ 3 mn ou plus), sans que le principe actif puisse pénétrer à l'intérieur du granule.

Avec le procédé qui fait l'objet de la présente invention, on obtient finalement des granules homéopathiques idéaux à porosité élevée et se dissolvant rapidement (dissolution suslinguale en 1 minute-1,5 minute).

Ce temps de dissolution peut être abaissé pratiquement à volonté, car la dureté, la porosité et la friabilité du granule sont réglables et variables. On peut donc créer un produit vraiment excellent pour l'homéopathie. Si c'est nécessaire pour habituer graduellement le public à la plus grande vitesse de dissolution, on pourra éventuellement doter les granules d'un enrobage de type classique pour ralentir la dissolution.

Comme on l'a déjà dit, il faut souligner encore une fois que le secteur homéopathique est seulement un des nombreux domaines d'emplois qui s'ouvrent pour les granules conformes à la présente invention. Ainsi, dans le secteur des substances actives pharmaceutiques, on pourra supprimer les neutres pour certains produits pour lesquels on désire obtenir un dosage élevé, en employant comme support des petits granules actifs obtenus avec ce procédé. En plus, on pourra obtenir des granules à action prolongée ou retardée et/ou microencapsulés, en une seule opération à la place des nombreux passages nécessaires avec les techniques actuelles.

Les pellets ou granules ainsi formés peuvent être considérés, en outre, comme des micro-éponges à porosité élevée et on pourra ainsi obtenir le même résultat de dissolution rapide du principe actif que l'on obtient actuellement avec la lyophilisation, avec des granules obtenus par le procédé qui fait l'objet de la présente invention, qui s'adapte, en outre, très bien en raison de sa flexibilité, aux caractéristiques de chaque matière première comme par exemple, les traitements à l'état fondu, les gouttes de solutions et de suspensions, etc...

On peut donc affirmer qu'avec la présente invention, s'ouvre un domaine vraiment immense pour l'emploi de granules comme formes de présentation des substances des qualités les plus diverses, et non pas seulement pharmaceutiques, mais aussi alimentaires, chimiques, etc...

Pour illustrer encore mieux la portée de l'invention, on décrira maintenant de façon détaillée, une forme de réalisation actuellement préférée mais toujours à titre d'exemple, non limitatif, d'un appareil pour la production de granules en mettant en œuvre le procédé de la présente invention, en référence aux dessins annexés qui sont purement illustratifs et non pas limitatifs, et dans lesquels :

la figure 1 est une vue schématique générale en élévation de l'appareil donnée à titre d'exemple, avec des parties en coupe et des parties supprimées, pour montrer ses caractéristiques structurelles ;

la figure 2 est une vue frontale de la partie de l'appareil de formation, de chute et de sélection des granules ;

la figure 3 est une vue très schématique de la batterie des buses distributrices de gouttes et de l'air de contrôle de ces gouttes.

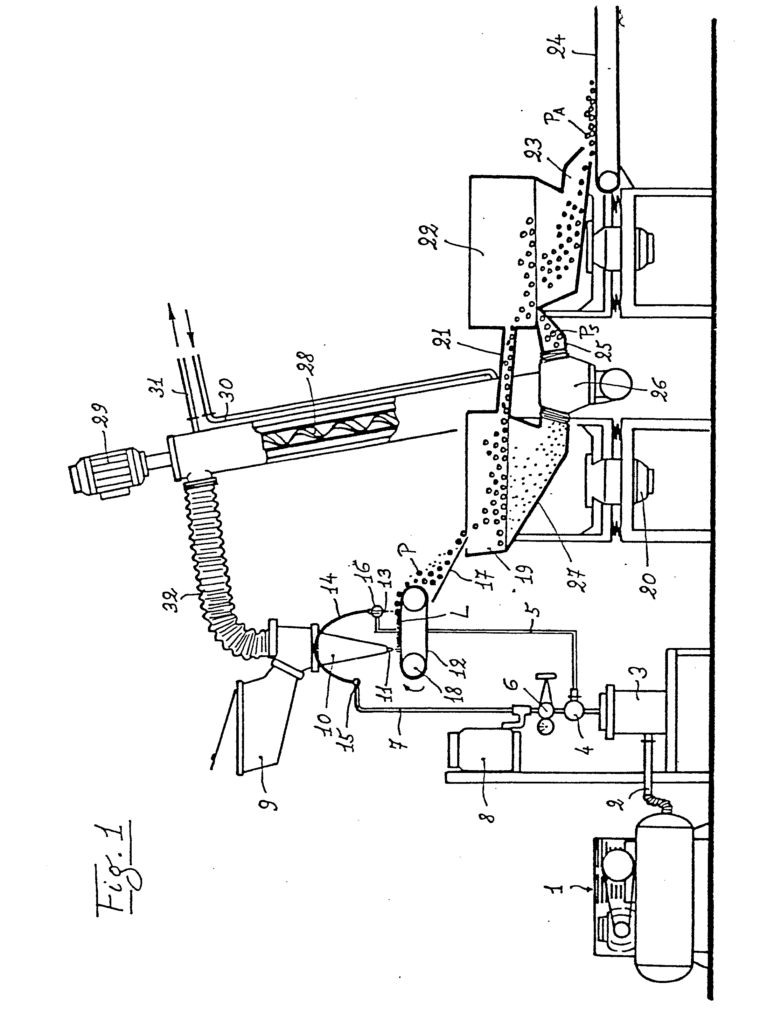

En faisant maintenant référence aux figures annexées dans lesquelles un même numéro de référence correspond à la même pièce, l'appareil préféré pour la réalisation du procédé selon la présente invention, comprend une série de groupes ou éléments qui dans les dessins annexés (en particulier dans la figure 1) sont montrés supportés sur des bâtis ou des châssis séparés, avant tout pour rendre plus simple et compréhensible le dessin, mais il est évident que tous ces groupes ou éléments peuvent être assemblés sur un châssis unique, formant ainsi un appareillage unitaire. Pour cette raison, les structures de support, comme elles peuvent être de n'importe quel type connu et ne requièrent pas de dispositions particulières nouvelles, ne sont pas décrites en détail et pourront être réalisées selon les exigences et les circonstances de la production, de l'emploi et de l'espace disponible.

L'appareil préféré pour la mise en œuvre du procédé selon · la présente invention comprend donc une source d'air comprimé qui peut être un compresseur autonome 1 comme désigné dans la figure 1, ou l'air comprimé du réseau central de l'établissement qui envoie l'air comprimé par l'intermédiaire d'une tuyauterie 2 à un filtre 3 d'élimination de l'humidité, d'où l'air passe, à travers une vanne de sécurité 4 jusqu'à une conduite 5 qui envoie l'air aux buses qui seront décrites plus loin, ainsi qu'à un manomètre régulateur de pression 6 qui réduit la pression à une très faible valeur (quelques millibars) et envoie l'air dans une tuyauterie 7 dans laquelle il sert de véhicule de transport et de poussée du liquide producteur de gouttes provenant du récipient 8.

La matière première pulvérulente, qui peut être une substance active du point de vue pharmaceutique ou neutre, est chargée par des moyens manuels, mécaniques ou automatiques, dans la trémie 9 d'où elle passe dans l'entonnoir 10 qui se termine à sa partie inférieure par une ouverture longitudinale 11 qui distribue une quantité constante et dosée de matière première sur la bande transporteuse sans fin 12, entraînée par les rouleaux 18 avantageusement motorisés et sur le brin supérieur de laquelle la matière première forme un lit ou tapis L sur lequel on laisse tomber les gouttes G qui sortent des aiguilles doseuses 13 fixées d'une façon amovible (elles sont interchangeables selon le liquide qui est employé pour former les gouttes) aux petits tuyaux 14, qui sont tous alimentés par le collecteur de distribution 15 placé à l'extrémité de la tuyauterie 7.

Les aiguilles 13 sont disposées de telle manière qu'elles passent à l'intérieur du collecteur de distribution 16 de l'air comprimé qui provient de la conduite 5 qui alimente le collecteur 16 à chacune de ses extrémités pour maintenir dans ce dernier une pression rigoureusement uniforme.

Les aiguilles 13 sont disposées chacune au centre d'une buse correspondante, coaxialement au collecteur 16, d'où elles avancent légèrement vers le bas ou au maximum elles se trouvent au même niveau que l'embouchure de la buse, d'une façon telle que la goutte G en sortant de l'aiguille est effleurée d'une façon homogène sur toute sa surface par un flux concentrique laminaire d'air qui en contrôle la forme et la grandeur, qui sont déterminées par la pression de l'air comprimé, par les dimensions de l'aiguille et de la buse, par la fréquence de fuite de la goutte et par les caractéristiques physico-chimiques du liquide.

Ces gouttes en tombant sur le lit L de matière première forment directement les granules P parfaitement sphériques dont la grandeur dépend une fois encore des caractéristiques physico-chimiques de la matière première, du liquide et de la dimension de la goutte, tandis que la vitesse de mouvement de la bande transporteuse 12 doit être réglée selon la fréquence de chute des gouttes, pour empêcher la superposition des gouttes et donc des granules P sur le lit L.

Les granules P ainsi formés ainsi que la matière première du lit L non employée tombent sur une gouttière à secousses ou sur une glissière métallique 17 vibrante, car elle est solidaire du tamis vibrant 19 qui est avantageusement mû par le moteur 20.

La matière première non employée et les éventuels granules trop petits passent à travers les mailles du tamis, tandis que les granules P qui ne passent pas à travers les mailles du tamis 19 sont déchargés par la sortie latérale 21 vers un deuxième tamis vibrant 22, dont les mailles laissent passer les granules ou pellets $P_A$ de diamètre acceptable qui, à travers la conduite de décharge 23 sont envoyés sur la bande transporteuse 24 ou à un autre système de collecte fixe ou mobile des granules $P_A$ prêts à l'emploi, tandis que les éventuels granules $P_s$ retenus sur le tamis 22, parce que de dimension trop grande, sont écartés par la sortie latérale 25 et sont renvoyés dans la trémie de chargement 26 dans laquelle arrivent également, par l'intermédiaire de la conduite de chargement 27 du premier tamis 19, la matière première non employée et les granules trop petits.

Cette matière première non employée ou écartée est recyclée et renvoyée à l'entonnoir 10 par l'intermédiaire d'une vis sans fin 28 mûe par un moteur 29. Comme la matière première a été mouillée par le liquide producteur de gouttes et qu'elle est en général hygroscopique, comme dans le cas du saccharose et lactose pour les neutres, il est nécessaire d'évaporer le liquide et de remettre la matière première à l'état pulvérulent car dans le cas contraire, elle formerait une pâte sur la vis sans fin 28 et la bloquerait. Pour éviter cela, la vis sans fin 28 est chauffée au moyen d'un système quelconque connu dans la technique. Dans l'appareil préféré représenté à la figure 1, ce chauffage est réalisé au moyen d'eau chaude en circulation autour de la vis sans fin dans une double enveloppe ou un serpentin dans les tuyaux d'aller et retour 30 et 31. De cette façon, la matière première complètement sèche et pulvérulente arrive au sommet de la vis sans fin 28 d'où elle est envoyée à l'entonnoir 10 par l'intermédiaire du tuyau 32.

L'appareil est généralement placé dans une chambre insonorisée et munie de moyens de ventilation pour évacuer la poudre nébulisée de la matière dans l'atmosphère ambiante. Cet appareil est maintenu dans une ambiance à faible luminosité pour permettre un meilleur contrôle visuel de la chute des gouttes au moyen de la lampe stroboscopique 33, disposée latéralement et parallèlement au collecteur 16.

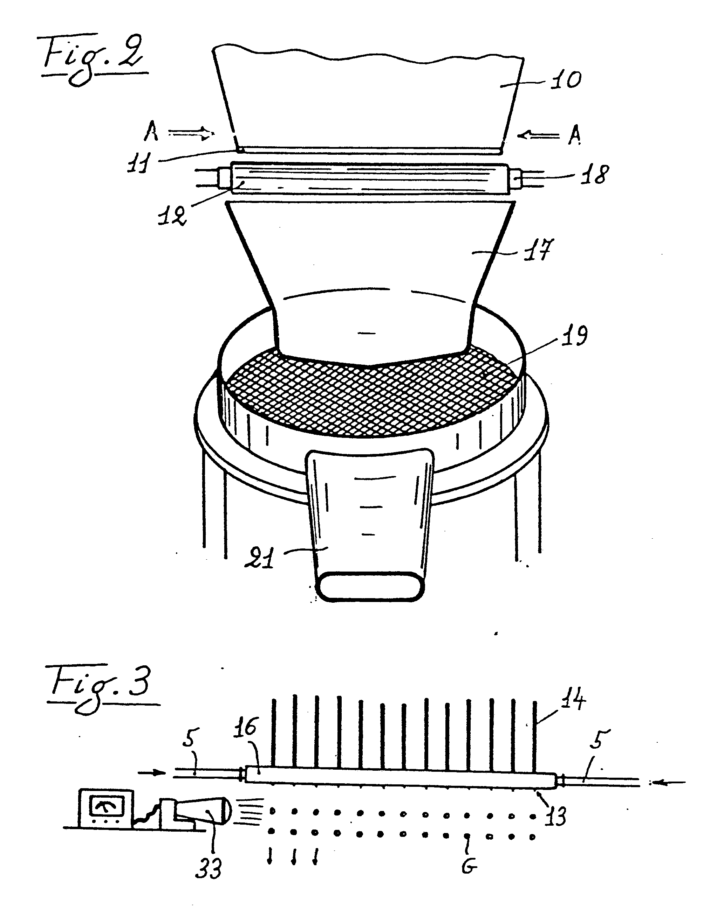

Pour permettre une meilleure compréhension des dessins, les éléments illustrés dans la figure 3 ont été montrés séparément, mais en observant la figure 1 et les flèches A-A de la figure 2, il est clair qu'ils sont disposés avant l'entonnoir 10, un peu plus haut que l'ouverture de distribution 11. Pour simplifier et rendre compréhensible le dessin, on a indiqué à la figure 3 un nombre limité de petits tuyaux 14 et d'aiguilles 13 correspondantes, relativement espacés les uns des autres, mais dans la pratique, la machine pourra présenter une batterie d'aiguilles très nombreuses et rapprochées, par exemple : 150 sorties, avec lesquelles on pourra arriver à des productions très élevées par exemple : 300 kgs/heure, ce qui est tout à fait impensable avec les méthodes actuelles, tout cela avec un espace réduit et un coût de fabrication extrêmement réduit vu sa simplicité et l'absence d'élément difficile à trouver ou construire.

## Revendications

1. Procédé pour la formation de granules parfaitement sphériques et poreux au moyen de la chute de gouttes d'un liquide sur un lit de matière pulvérulente, caractérisé par le fait que la forme et la dimension des gouttes émises individuellement sont contrôlées par un flux d'air laminaire et concentrique à la goutte, qui effleure la surface de la goutte quand celle-ci est émise.

2. Procédé selon la revendication 1, caractérisé par le fait que le lit de matière pulvérulente est en mouvement continu et est alimenté en continu, soit par l'introduction de matière fraîche, soit par recyclage de la matière non utilisée pour former des granules et des granules non conformes à la gamme de dimensions prévue.

3. Procédé selon la revendication 2, caractérisé par le fait que la matière à recycler est chauffée et séchée pour qu'elle redevienne parfaitement sèche et pulvérulente avant son réemploi.

4. Procédé selon l'une quelconque des revendications 1 à 3, caractérisé par le fait que l'air de contrôle des gouttes est sous pression et une partie de cet air est employée avec un régulateur de pression pour alimenter le liquide producteur de gouttes.

5. Procédé selon l'une quelconque des revendications 1 à 4, caractérisé par le fait que les granules utilisables sont sélectionnés et séparés de la matière pulvérulente non utilisée et des granules qui n'entrent pas dans la gamme de dimensions prévue au moyen de deux opérations de criblage successives.

6. Appareil pour la formation de granules parfaitement sphériques et poreux en mettant en œuvre le processus selon l'une quelconque des revendications 1 à 5, caractérisé par le fait qu'il comprend substantiellement des moyens d'alimentation de la matière première, des moyens pour créer et déterminer l'écoulement d'un lit ou tapis de matière première au-dessous des moyens distributeurs de gouttes, à partir d'un liquide de production des gouttes, des moyens d'alimentation du liquide producteur de gouttes, des moyens d'alimentation et de distribution d'air sous pression autour des moyens distributeurs de gouttes, des moyens pour sélectionner et transporter les granules produits et des moyens pour recycler la matière première et les granules éliminés par les moyens de sélection.

7. Appareil selon la revendication 6, caractérisé par le fait que les moyens d'alimentation de la matière première sont constitués par une trémie de chargement et par un entonnoir qui se termine à sa partie inférieure par une fissure longitudinale d'écoulement.

8. Appareil selon l'une quelconque des revendications 6 et 7, caractérisé par le fait que le lit de matière première est créé et son écoulement est déterminé au moyen d'une bande transporteuse sans fin, en mouvement continu sur laquelle la matière première est distribuée par les moyens d'alimentation.

9. Appareil selon la revendication 6, caractérisé par le fait que le liquide producteur de gouttes est amené, sous une pression d'air réglée aux moyens distributeurs de gouttes, constitués par une pluralité d'aiguilles doseuses interchangeables fixées de façon amovible aux extrémités d'un nombre correspondant de petits tuyaux qui sont reliés à l'alimentation de liquide.

10. Appareil selon les revendications 6 et 9, caractérisé par le fait que les moyens d'alimentation et de distribution d'air sous pression sont constitués par un collecteur qui reçoit de l'air sous pression à ses deux extrémités et émet cet air par une série de buses disposées coaxialement autour de chaque aiguille de distribution du liquide pour contrôler les gouttes grâce au flux d'air laminaire et concentrique ainsi émis.

11. Appareil selon la revendication 6, caractérisé par le fait que les moyens pour sélectionner et transporter les granules produits, sont constitués par un premier crible ou tamis qui sépare les granules au-dessus d'un certain diamètre des granules trop petits et de la matière première non utilisée et les envoie à un deuxième crible ou tamis qui sépare les granules qui sont à la dimension désirée des granules qui sont trop gros et les envoie aux moyens de collecte et de transport en vue de leur emploi.

12. Appareil selon les revendications 6 et 11, caractérisé par le fait que la matière première non employée et les granules éliminés sont recyclés au moyen d'une vis sans fin qui les ramène aux moyens d'alimentation de la matière première.

13. Appareil selon la revendication 12, caractérisé par le fait que la vis sans fin est munie de moyens de chauffage pour oter l'humidité et sécher la matière à recycler afin de la rendre à nouveau sèche et pulvérulente.

14. Appareil selon la revendication 13, caractérisé par le fait que les moyens de chauffage de la vis sans fin sont constitués par une circulation d'eau chaude autour de cette vis à l'intérieur d'une double enveloppe ou d'un serpentin alimenté par un circuit fermé ou ouvert.

15. Appareil selon l'une quelconque des revendications 6 à 14, caractérisé par le fait que la chute des gouttes est contrôlée visuellement au moyen d'une lampe stroboscopique disposée latéralement aux gouttes et parallèlement à la série des moyens distributeurs de gouttes.

## Claims

1. Process for the production of perfectly spherical and porous granules by means of the dropping of droplets of a liquid onto a bed of powdered material, characterized by the fact that the shape and size of the droplets individually emitted are controlled by a laminar air flow concentric with the droplet, which skims the surface of the droplet when the latter is emitted.

2. Process according to claim 1, characterized by the fact that the bed of powdered material is in continuous motion and is supplied continuously, either by the production of fresh material, or by recycling the material unused to form granules and granules not conforming to the specified range of dimensions.

3. Process according to claim 2, characterized by the fact that the material to be recycled is heated and dried so that it again becomes perfectly dry and powdery before its reuse.

4. Process according to any one of claims 1 to 3, characterized by the fact that the air for controlling the droplets is under pressure and a portion of this air is employed with a pressure regulator to supply the liquid producing droplets.

5. Process according to any one of claims 1 to 4, characterized by the fact that the useful granules are selected and separated from the unused powdered material and from the granules which do not come within the range of dimensions specified by means of two successive sifting operations.

6. Apparatus for the production of perfectly spherical and porous granules employing the process according to any one of claims 1 to 5, characterized by the fact that it comprises substantially means for supplying raw material, means for creating and determining the flow of a bed or belt of raw material beneath the droplet distributing means, from a droplet-producing liquid, means for supplying droplet-producing liquid, means for supplying and distributing pressurized air around the droplet distributing means, means for selecting and transporting the granules produced and means for recycling the raw material and the granules eliminated by the selection means.

7. Apparatus according to claim 6, characterized by the fact that the supply means for the raw material are constituted by a charging hopper and by a funnel which is terminated at its lower part by a longitudinal flow slot.

8. Apparatus according to any of claims 6 and 7, characterized by the fact that the bed of raw material is created and its flow is determined by means of an endless conveyor belt, in continuous motion onto which the raw material is distributed by the supply means.

9. Apparatus according to claim 6, characterized by the fact that the droplet-producing liquid is brought under a regulated air pressure to the droplet-distributing means, constituted by a plurality of interchangeable metering needles movably fixed to the ends of a corresponding number of small ducts which are connected to the liquid supply.

10. Apparatus according to claims 6 and 9, characterized by the fact that the means for supplying and distributing pressurized air are constituted by a manifold which receives air under pressure at its two ends and sends this air through a series of nozzles arranged coaxially around each liquid-distributing needle for controlling the droplets by means of the laminar and concentric flow of air thus emitted.

11. Apparatus according to claim 6, characterized by the fact that the means for selecting and transporting the granules produced are constituted by a first sieve or screen which separates the granules above a certain diameter from granules which are too small and from the unused raw material and sends them to a second sieve or screen which separates the granules which are of the desired dimension from the granules which are too coarse and sends them to collecting and transporting means for their utilization.

12. Apparatus according to claims 6 and 11, characterized by the fact that the unused raw material and the granules eliminated are recycled by means of a worm-screw which brings them back to the supply means for the raw material.

13. Apparatus according to claim 12, characterized by the fact that the worm-screw is provided with heating means to remove the moisture and dry the material to be recycled in order to render it dry and powdery again.

14. Apparatus according to claim 13, characterized by the fact that the heating means of the worm-screw are constituted by a flow of hot water around this screw inside a double jacket or coil supplied through a closed or open circuit.

15. Apparatus according to any one of claims 6 to 14, characterized by the fact that the dropping of the droplets is controlled visually by means of a stroboscopic lamp placed laterally to the droplets and parallel with the series of droplet-distributing means.

**Patentansprüche**

1. Verfahren zur Ausbildung von vollkommen sphärischen und porösen Kügelchen mittels Herabfallenlassen von Tropfen einer Flüssigkeit auf ein Bett aus pulverförmigem Material, dadurch gekennzeichnet, daß die Form und die Dimension der einzeln ausgestoßenen Tropfen durch einen laminaren und zum Tropfen konzentrischen Luftfluß gesteuert wird, der die Oberfläche des Tropfens berührt, wenn dieser ausgestoßen wird.

2. Verfahren nach Anspruch 1, dadurch gekennzeichnet, daß das Bett aus pulverförmigem Material in fortgesetzter Bewegung ist und fortgesetzt gespeist wird, und zwar entweder durch Einführen von frischem Material oder durch Wiederverwertung des Materials, das zum Ausbilden von Kügelchen nicht verbraucht worden ist, und von Kügelchen, die dem vorgesehenen Dimensionsbereich nicht entsprechen.

3. Verfahren nach Anspruch 2, dadurch gekennzeichnet, daß das wieder zu verwertende Material erwärmt und getrocknet wird, damit es vor seiner Wiederverwendung wieder vollkommen trocken und pulverförmig wird.

4. Verfahren nach einem der Ansprüche 1 bis 3, dadurch gekennzeichnet, daß die Luft zum Steuern der Tropfen unter Druck ist und ein Teil dieser Luft mit einem Druckregulator zum Zuführen der tropfenerzeugenden Flüssigkeit verwendet wird.

5. Verfahren nach einem der Ansprüche 1 bis 4, dadurch gekennzeichnet, daß die verwendbaren Kügelchen von dem nicht verbrauchten Ausgangsmaterial und den Kügelchen, welche nicht in den vorgesehenen Dimensionsbereich fallen, mittels zweier aufeinanderfolgender Aussiebungsvorgänge ausgewählt und abgetrennt werden.

6. Einrichtung zur Ausbildung von vollkommen sphärischen und porösen Kügelchen zum Durchführen des Verfahrens nach einem der Ansprüche 1 bis 5, dadurch gekennzeichnet, daß sie im wesentlichen Mittel zur Zuführung des Ausgangsmaterials, Mittel zum Erzeugen und Festsetzen des Ausfließens eines Betts oder Teppichs aus Ausgangsmaterial unter Mitteln zum Verteilen von Tropfen aus einer tropfenerzeugenden Flüssigkeit, Mittel zur Zuführung der tropfenerzeugenden Flüssigkeit, Mittel zur Zuführung und Verteilung von Luft unter Druck um die Mittel zum Verteilen von Tropfen, Mittel zur Auswahl und zum Transportieren der erzeugten Kügelchen und Mittel zum Wiederverwerten des Ausgangsmaterials und der Kügelchen, die durch die Mittel zur Auswahl ausgeschieden worden sind, umfaßt.

7. Einrichtung nach Anspruch 6, dadurch gekennzeichnet, daß die Mittel zur Zuführung des Ausgangsmaterials von einem Ladetrichter und von einem Trichter, der in seinem unteren Teil in einem Längsschlitz für das Ausfließen endet, gebildet sind.

8. Einrichtung nach einem der Ansprüche 6 und 7, dadurch gekennzeichnet, daß mittels eines in fortlaufender Bewegung befindlichen endlosen Transportbandes, auf dem das Ausgangsmaterial durch die Mittel zur Zuführung verteilt wird, das Bett aus Ausgangsmaterial erzeugt und sein Ausfließen festgelegt wird.

9. Einrichtung nach Anspruch 6, dadurch gekennzeichnet, daß die tropfenerzeugende Flüssigkeit unter einem regulierten Luftdruck zu den Mitteln zum Verteilen von Tropfen zugeführt wird, die von einer Mehrzahl von austauschbaren Dosiernadeln gebildet sind, welche in auswechselbarer Weise an den Enden eine entsprechende Anzahl von kleinen Leitungen, die mit der Zuführung der Flüssigkeit verbunden sind, befestigt sind.

10. Einrichtung nach den Ansprüchen 6 und 9, dadurch gekennzeichnet, daß die Mittel zur Zuführung und zum Verteilen von Luft unter Druck von einem Sammelkanal gebildet sind, der die Luft unter Druck an seinen beiden Enden erhält und diese Luft durch eine Reihe von Düsen ausstößt, die koaxial um jede Nadel zur Verteilung der Flüssigkeit zum Steuern der Tropfen aufgrund des so ausgestoßenen laminaren und konzentrischen Luftflusses vorgesehen sind.

11. Einrichtung nach Anspruch 6, dadurch gekennzeichnet, daß die Mittel zur Auswahl und zum Transportieren der erzeugten Kügelchen von einem ersten Sortierer oder Sieb gebildet sind, der bzw. das die Kügelchen oberhalb eines gewissen Durchmessers von den zu kleinen Kügelchen und dem nicht verbrauchten Ausgangsmaterial abtrennt und sie zu einem zweiten Sortierer oder Sieb schickt, der bzw. das die Kügelchen, welche die gewünschte Dimension haben, von den Kügelchen, die zu groß sind, abtrennt und sie

mit Mitteln des Sammelns und des Transports zum Zwecke ihrer Anwendung wegschickt.

12. Einrichtung nach den Ansprüchen 6 und 11, dadurch gekennzeichnet, daß das nicht verbrauchte Ausgangsmaterial und die ausgeschiedenen Kügelchen mittels einer Endlosschnecke wiederverwertet werden, die sie wieder zu den Mitteln zur Zuführung des Ausgangsmaterials bringt.

13. Einrichtung nach Anspruch 12, dadurch gekennzeichnet, daß die Endlosschnecke mit Mitteln zur Erwärmung zum Wegnehmen der Feuchtigkeit und zum Trocknen des wiederzuverwertenden Materials versehen ist, um es von neuem trocken und pulverförmig zu machen.

14. Einrichtung nach Anspruch 13, dadurch gekennzeichnet, daß die Mittel zum Erhitzen der Endlosschnecke von einer Zirkulation von Warmwasser um diese Schnecke im Inneren eines Doppelmantels oder einer Schlange, der bzw. die durch einen geschlossenen oder offenen Kreis gespeist wird, gebildet sind.

15. Einrichtung nach einem der Ansprüche 6 bis 14, dadurch gekennzeichnet, daß das Herabfallen der Tropfen visuell mittels einer stroboskopischen Lampe kontrolliert wird, die seitlich von den Tropfen und parallel zu der Reihe der Mittel zum Verteilen der Tropfen vorgesehen ist.

Fig. 1

*Fig.2*

*Fig.3*